# EUROPEAN PATENT SPECIFICATION

(11) **EP 1 765 342 B1**
(45) Date of publication and mention of the grant of the patent: **03.11.2010**
(21) Application number: 05751188.3
(22) Date of filing: 09.06.2005
(51) Int. Cl.: A61K 31/4422, A61K 31/401

(54) **PHARMACEUTICAL COMPOSITION COMPRISING AMLODIPINE BESILATE AND LISINOPRIL DIHYDRATE AND PROCESS FOR THE PREPARATION THEREOF**
PHARMAZEUTISCHE ZUSAMMENSETZUNG MIT AMLODIPINBESILAT UND LISINOPRILDIHYDRAT UND HERSTELLUNGSVERFAHREN DAFÜR
COMPOSITION PHARMACEUTIQUE COMPRENANT DU BESYLATE D' AMLODIPINE ET DU LISINOPRIL DIHYDRATE ET DONT METHODE DE PREPARATION.

(30) Priority: 10.06.2004 HU 0401170
(43) Date of publication of application: 28.03.2007
(73) Proprietor: Richter Gedeon Nyrt., 1103 Budapest (HU)
(72) Inventor: KELEN, Ákos, H-1124 Budapest (HU)
(74) Representative: HOFFMANN EITLE
(86) International application number: PCT/HU2005/000064
(87) International publication number: WO 2005/120502

(56) References cited:
- WO-A-96/28185
- MITAL S ET AL: "Synergy of amlodipine and angiotensin-converting enzyme inhibitors in regulating myocardial oxygen consumption in normal canine and failing human hearts" AMERICAN JOURNAL OF CARDIOLOGY, CAHNERS PUBLISHING CO., NEWTON, MA,, US, vol. 83, no. 12A, 17 June 1999 (1999-06-17), pages 92H-98H, XP002339217 ISSN: 0002-9149
- ANGYAL, NORA ET AL: "Formulation of a capsule preparation with high active ingredient content using recrystallized drug compound" ACTA PHARMACEUTICA HUNGARICA , 74(1), 45-50 CODEN: APHGAO; ISSN: 0001-6659, 2004, XP001207323

## Description

The present invention relates to a new pharmaceutical composition and a process for the preparation thereof.

More particularly, the present invention relates to a pharmaceutical composition containing a combination of two active ingredients, namely 3-Ethyl 5-methyl (4*RS*)-2-[(2-aminoethoxy)methyl]-4-(2-chlorophenyl)-6-methyl-1,4--dihydro-pyridine-3,5-dicarboxylate benzenesulphonate, further referred to as amlodipine besilate (CAS No.: 83915-83-7) [c.Ph.Eur..] and (S)-1-[N²-(1-carboxy-3-phenylpropyl)-L-lysyl]-L-proline-dihydrate, further referred to as lisinopril dihydrate (CAS No.: 111470-99-6; No. of the free base 88150-42-9) [c.Ph.Eur. and c.USP]. The invention also presents an advantageous method for the production of the new pharmaceutical composition.

It is known that the combination of active pharmaceutical ingredients selected from amlodipin or its pharmaceutically acceptable salts and an ACE inhibitor e.g. lisinopril is beneficial to prevent and/or treat cardiovascular diseases, see inter alia U.S. Pat. No. 6,245,787 and published Hungarian patent application No. HU02 02416. Clinical data are known in the art, but neither the constituents of the pharmaceutical composition used nor the method for the production thereof are mentioned.

It is known that compositions containing amlodipine besilate as sole pharmaceutically active ingredient contain microcrystalline cellulose, calcium hydrogen phosphate, magnesium stearate, sodium carboxymethyl amylopectin and optionally colouring agents as excipients [inter alia Norvasc tablets, see PDR 58, 2004].

It is also known that compositions containing lisinopril as sole pharmaceutically active ingredient contain calcium hydrogen phosphate, starch, magnesium stearate, mannitol optionally colouring agents as excipients [inter alia Privinil, Prinzide, Zestril tablets, see PDR 58, 2004 and Acerbon, Coric, Lisihexal, Lisinopril-ratiopharma tablets, see Rote Liste, 2003].

A composition containing both of amlodipine besilate and lisinopril dihydrate as pharmaceutically active ingredients and containing calcium phosphate, magnesium stearate, microcrystalline cellulose and optionally colouring agents as excipients is known [http://203.115.112.137:8080/ipca/ipca-corp/index.html, 2004].

All the above mentioned excipients are well known and widely used for decades in the pharmaceutical industry. The calcium salt of phosphoric acid is present in each disclosed composition, being excellent for the formulation of moisture-sensitive drugs [Lachman, L. et al.: The Theory and Practice of Industrial Pharmacy, pp. 326., 1986]. The calcium phosphate is known as a non hygroscopic excipient which does not become hydrated even in the case of great relative humidity [Schmidt, P.S. and Hercog, I.L.: Calcium phosphate in pharmaceutical tableting. Pharm. World. Sci. 15(3) pp. 105-115., 1993]. This beneficial property, however, restricts its use: it functions simply as a filler and has no other adjuvant role. It has been found that when calcium phosphate is used as a sole filler, even in the presence of a glidant and/or lubricant friability and/or capping of the tablets may occur. Therefore its use as a single filler is not typical [Kachrimanis, K. and Malamataris, S.: Apparent Young's elastic modulus and radial recovery for some tableted pharmaceutical excipients. Eur. J. Pharm. Sci. 21; pp. 197-207., 2004]. The additional beneficial properties of calcium phosphate are the excellent flowability and outstanding stability that makes it suitable for the formulation of active agents characterised by unsatisfactory flow and/or insufficient stability, especially, moisture-sensitivity [Banker, G.S. and Rhodes C.T.: Modem Pharmaceutics, Marc Dekker, New York, pp. 364., 1990]. The hitch in the case of the formulation of lisinopril dihydrate as pharmaceutically active ingredient with calcium phosphate is the difference in bulk densities. The characteristic bulk density of calcium phosphate is far greater than the characteristic bulk density of lisinopril dihydrate.

In case of a new pharmaceutical composition the simplest technology should be tried first that requires the possible least equipment and operational steps. It is the simple homogenization followed by direct compression or capsule filling [Remington: The Science and Practice of Pharmacy chapter 37. and 45., 2000]. The simple homogenization is irreproducible and not easy to carry out when there are great differences in the particle size and/or shape and/or density of the different materials. It is essential to meet the requirements of the "uniformity of content of single-dose preparations" test [c.Ph.Eur. chapter 2.9.6., 2004 and c.USP chapter 905., 2004]. The bad flow and homogenization properties of the materials deriving from particle features, can be overcome by the agglomeration techique.

When the two different pharmaceutically active ingredients (namely amlodipine besilate and lisinopril dihydrate) were formulated to form one composition the following difficulties came up:
1. The lisinopril dihydrate is characterised by small particle size (< 40 µm min. 80 % and < 125 µm min. 95 %), needle-shaped particles and small bulk density (approximately 0.05-0.30 g/ml). All the above mentioned features-cause inopportune flow and compressibility etc. even if it is present in minimal proportion that ensures therapeutic effect and may endanger the required homogeneity, compressibility and/or capsule filling even after dry granulation. The addition of glidants, lubricants and diluents in pharmaceutically acceptable proportion does not provide the appropriate bulk properties. In this case wet granulation can be a solution.
2. The difference between the particle sizes and bulk densities of amlodipine besilate and lisinopril dihydrate are great thus the proper homogenization of the two active ingredients is rather questionable (the particle size distribution of lisinopril dihydrate is given above, the particle size of amlodipine besilate is < 45 µm min. 50 % and < 90 µm min. 90 % and its bulk density is approximately 0.50-0.80 g/ml).
3. On heating the lisinopril dihydrate undergoes dehydration first to the monohydrate (around 60-70°C) and then to the dehydrated form (around. 80-90°C) [Brittain, H.G.: Analytical profiles of drug substances and excipients, Volume 21., pp. 234-276., Academic Press Inc., 1992] which do not correspond to the form given in the c.Ph.Eur. and c.USP. For that very reason during the pharmaceutical formulation any production step that may promote dehydration should be avoided. Therefore the fluidization drying - commonly used after the wet granulation step - can only be safely applied when the air used has low temperature, white the moisture-content is not low. The drawback of the drying with such limited parameters is that it is extremely time-consuming [Handbook of pharmaceutical granulation technology, Marcel Dekker pp. 246., 1997 and Remington: The Science and Practice of Pharmacy pp. 865., 2000].
4. A known degradation product of lisinopril dihydrate is S,S,S-diketopiperazine [c.Ph.Eur. and c.USP]. The degradation process starts at 40-50°C, thus the drying step should take place under this critical temperature. The limited parameters result in that the drying procedure is extremely time-consuming.
5. The known degradation product of amlodipine besilate is the oxidated amlodipine besilate [c.Ph.Eur.]. Oxidizers, UV light, simultaneous effect of high temperature and moisture may accelerate the degradation process thus these conditions should be avoided. Simple homogenization followed by direct compression or capsule filling can be suitable.
6. A mixture of amlodipine besilate and lisinopril dihydrate after being wetted with about 10 wt % of water was stored above room temperature (at 40-50 °C). An increase in the total amount of the decomposition products was observed compared with the amounts found when the active ingredients were stored separately under the same conditions, showing that an incompatibility may exist in the case of such formulation. Therefore wet granulation followed by the commonly used fluidization drying is not a suitable formulation technology in case of this combination.

Regarding all the above mentioned data and difficulties the aim of the present invention is to elaborate a procedure that is suitable for the preparation of a composition containing the two active ingredients. Said procedure should ensure the reproducible industrial production of the product that satisfies all the strict pharmaceutical regulatory, stability and safety demands as well as the environmental expectations.

The ascertained pharmaceutical incompatibility, heat and humidity-sensitivity query the possibility whether the two active ingredients can be successfully granulated/formulated together. The particle shape and size distribution of amlodipine besilate however, may make possible to homogenize it as a part of the outer phase to the lisinopril dehydrate being in the inner phase. The granulation of lisinopril dihydrate can hardly be avoided.

In our experiments wet granulation was found to be suitable to achieve the required particle size distribution of lisinopril dihydrate. To ensure the required moisture content of the final granule drying after the granulation should be carried out below the atmospheric pressure to reduce the boiling point of the granulation liquid and thereby also the product temperature to prevent the dehydratation of the active ingredient. Drying procedure can be accelerated via convection heating and/or dielectric heating [Lucisano und Poska: Microwave technology, Pharmaceutical Technology pp. 38-42., Apr. 1990]. Dielectric (microwave) heating is highly recommended because of its known selectivity. It means that the different components of the mixture to be dried absorb different amounts of electromagnetic energy, thus the temperature of the active agents can be lower than those of the auxiliaries. It is also known that the free water absorbs more energy than the bound water thus the free granulation liquid takes up heat more rapidly than the water in lisinopril dihydrate. Consequently the temperature of the active molecules can be lower than that of the ambient medium [Smet P.D. Vacuum drying; Manufacturing Chemistry, pp. 39., May 1989 and Meredith R.: Engineers' handbook of industrial microwave heating pp. 19-51., 1998]. If vacuum drying accelerated by dielectric energy is used the required moisture content of the granule can be ensured without the risk of decomposition and/or dehydration of the active agent(s), resulting in that the stability of the product - even when prepared without calcium phosphate - is surprisingly at least as good as that of the one prepared conventionally with calcium phosphate. In the case of scale up the dielectric drying is, again beneficial, since it is independent of the volume/surface ratio in contrast with the convection drying [Poska: Integrated mixing granulation and microwave drying, Pharmaceutical Engineering Vol. 11. No. 1, 1991].

Based on our experiments the following formulation technology has been elaborated to yield a pharmaceutical composition with the required purity and stability:

The active ingredients can be mixed with lactose, cellulose, starch, sucrose, mannitol, sorbitol, and calcium sulphate as commonly used diluents before the following step. The microcrystalline cellulose functions not only as a diluent; it has also some lubricant and disintegrant properties that make it beneficial. Carboxymethylcellulose calcium, carboxymethylcellulose sodium, croscarmellose sodium, crospovidone, starched and sodium starch glycolate can be added among others as disintegrants; starches, gelatin, hydroxypropyl cellulose, hydroxypropyl methylcellulose and povidone can be used among others as binders; and other excipients can be added to modify the solubility and/or release of the active ingredients.

If the inner phase contains only one of the active ingredients, preferably the lisinopril dihydrate, in admixture with some of the above mentioned excipient or excipients, then the other active ingredient can be admixed even with further excipients as a part of the outer phase before the compression or capsule filling step.

The present invention relates to a pharmaceutical composition containing 0.3-30 wt %(m/m) of amlodipine besilate and 0.1-75 wt %(m/m) of lisinopril dihydrate as active agents in admixture with one or more excipient(s) commonly used in the pharmaceutical industry, with the proviso that the excipient is other than a calcium salt of phosphoric acid.

In a preferred embodiment of the invention the ratio of amlodipine besilate and lisinopril dihydrate is 1:2, preferably 0.00694 g of amlodipine besilate and 0.01088 g of lisinopril dehydrate are present. As for the excipients 20-90 wt %(m/m) of microcrystalline cellulose, 2-8 wt %(m/m) of sodium carboxymethyl amylopectin and not more than 5 wt %(m/m) of magnesium stearate are prefered.

The present invention also relates to a process for the preparation of the above pharmaceutical composition, which comprises preparing a mixture of lisinopril dihydrate, sodium carboxymethyl amylopectin and microcrystalline cellulose, granulating said mixture with water, drying the wet granulated mass in vacuum at a product temperature below 35-40 °C, homogenizing the dry blend obtained with amlodipine besilate, sodium carboxymethyl amylopectin, and microcrystalline cellulose, finally mixing it with magnesium stearate and compressing the mixture into tablets or filling it into capsules.

According to a preferred embodiment of the invention first a mixture containing only the lisinopril dihydrate is granulated. The granulation be take place in a fluidizer or high shear equipment. If desired the blend can be sized, then the formulation is continued and the composition is dried below 40 °C at atmospheric or at reduced pressure. The drying process can be accelerated with the use of convection and/or preferably dielectric energy.

The dry blend can be sized again e.g. on sieve. If the blend has been already granulated then the granulation carried out after the drying is a so-called re-granulation step.

If necessary, additional excipients e.g. colloidal silicon dioxide, talc, calcium stearate, glyceryl monostearate, magnesium stearate, polyethylene glycol, sodium stearyl fumarate, stearic acid and zinc stearate are added as lubricants or glidants and/or different colouring and/or flavouring agents and/or additives modifying the drug release can be used.

The compressed tablets or filled capsules can be film or sugar-coated.

The composition obtained by the process according to the invention meets all the pharmaceutical requirements and the process is robust and reproducible both on pilot (20-50 kg) and plant scale (200-500 kg).

The stability test is carried out according to the ICH Harmosised Tripartite Guideline Stability Testing of New Drug Substance and Products (1998).

In Table 1 stability data of the invented composition are compared with the stability data of other known compositions containing amlodipine besilate and/or lisinopril dihydrate.

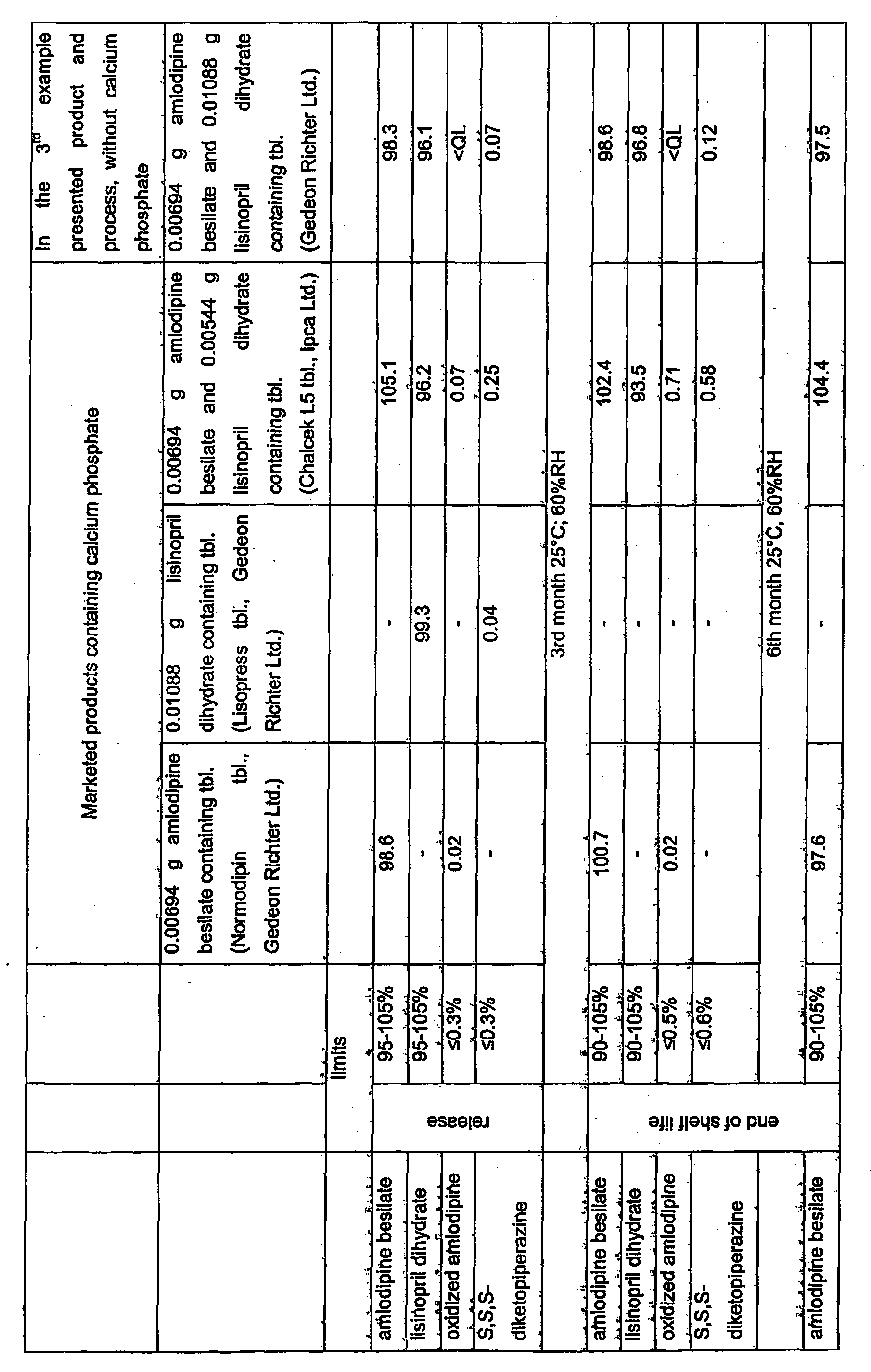

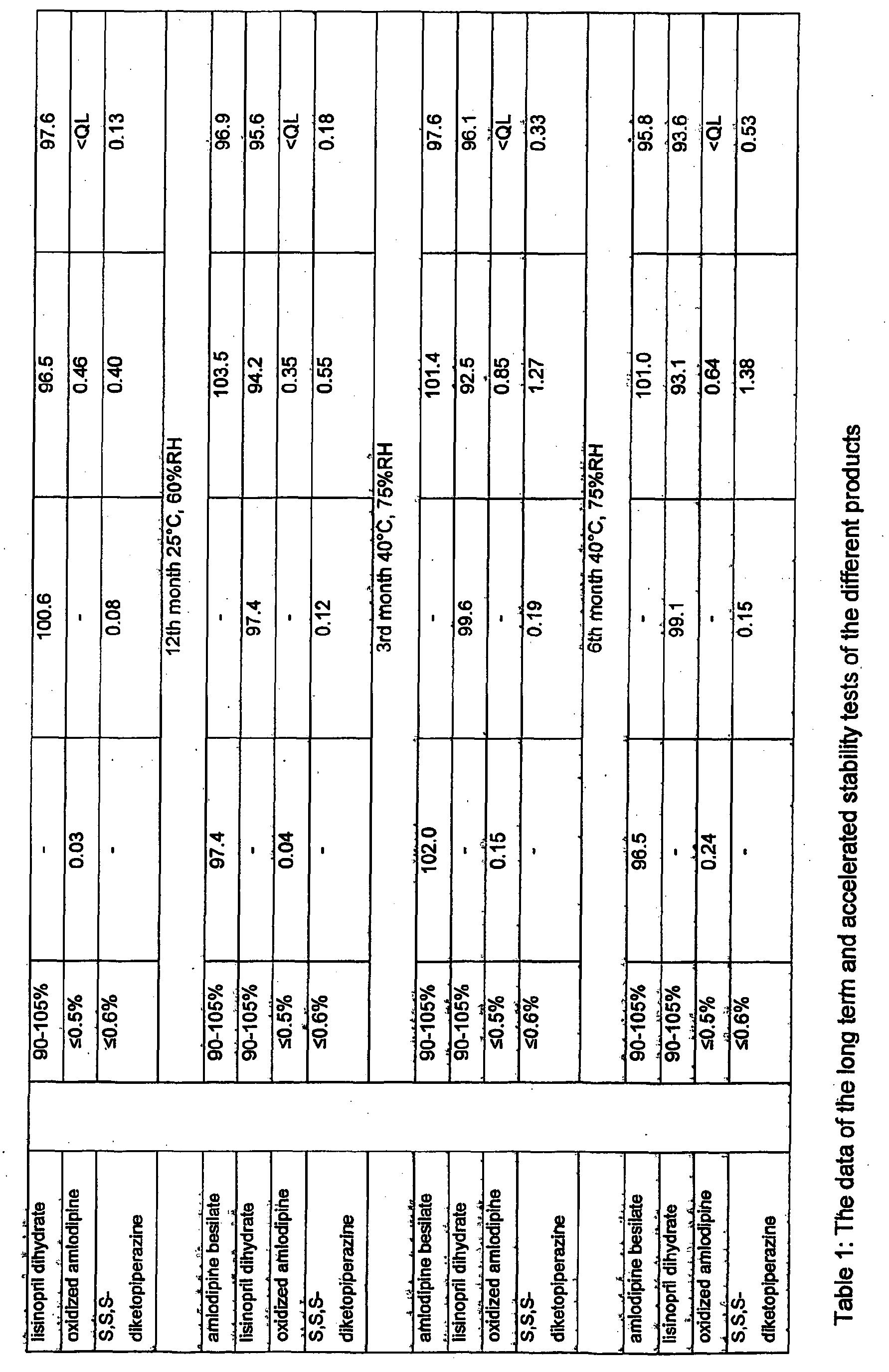

Comparing the stability data of the compositions containing amlodipine besilate (see Table 1.) it can be stated that, the composition produced by the novel process contains much lower amount of the degradation product, (namely oxidized amlodipine) than the products on the market.

The extent of degradation of lisinopril dihydrate is the lowest when the composition contains only lisinopril dihydrate and calcium phosphate. Comparing the compositions containing both active ingredients, in the composition prepared by the novel process of the invention, the amount of the S,S,S-diketopiperazine (degradation product) is less than the half of that found in the known product containing the two active agents formulated with calcium hydrogen phosphate.

It can be stated that the process according to the invention is surprisingly suitable for the formulation of both active ingredients even without calcium phosphate.

The invention is further illustrated by the following non-limiting Examples:

### Example 1.

4896.0 g of lisinopril dihydrate and 1171.125 g of amlodipine besilate are mixed with 1125.0 g of sodium starch glycolate, 20000.0 g of microcrystalline cellulose and 450.0 g of povidone. The mixture is granulated in a high shear mixer with 8500 g of purified water. (The binder can also be added as an aqueous polymer solution). The wetted mass is dried at a pressure 1-500 mbar until the required moisture content is reached. First 16615.375 g of MCC, than 675.0 g of magnesium stearate and 67.5 g of colloidal silicon dioxide are admixed. "Finally tablets are compressed. The composition of a tablet (0.4 g/tbl.) is as follows:

| | |
|---|---|
| amlodipine besilate | 0.01041 g |
| lisinopril dihydrate | 0.04352 g |
| colloidal silicon dioxide | 0.00060 g |
| povidone | 0.00400 g |
| magnesium stearate | 0.00600 g |
| sodium starch glycolate | 0.01000 g |
| microcrystalline cellulose | 0.32547 g |
| | 0.40000 g |

The dissolution of the product meets the requirements of Ph.Eur.

### Example 2.

The product is produced according to example 1, except that a fluidizer is applied in the process.

The dissolution of the product meets the requirements of Ph.Eur.

### Example 3.

2176.0 g of lisinopril dihydrate is mixed with 500.0 g of sodium starch glycolate, 9444.0 g of microcrystalline cellulose and 7100 g of purified water in a high shear equipment. The granule is dried at 10-100 mbar pressure. The drying process can be speeded up with heat convection and/or with radiation of dielectric energy to keep the product temperature below 35-40 °C. The dry granule is sieved (0.8 mm mesh), then homogenised with 1388.0 g of amlodipine besilate, 300.0 g of sodium starch glycolate and 25992.0 g of microcrystalline cellulose. It is finally mixed with 200.0 g of magnesium stearate and from the mixture tablets are compressed or filled, into capsules.

| | |
|---|---|
| amlodipine besilate | 0.00694 g |
| lisinopril dihydrate | 0.01088 g |
| magnesium stearate | 0.00100 g |
| sodium starch glycolate | 0.00400 g |
| microcrystalline cellulose | 0.17718 g |
| | 0.20000 g |

The dissolution of the product meets the requirements of Ph:Eur.

### Example 4.

The process described in example 3 is performed, with the alteration that a mixture of 544.0 g of lisinopril dehydrate, 500.0 g of sodium starch glycolate and 9444.0 g of microcrystalline cellulose is granulated with 1200.0 g of povidone in 5000 g of polymer aqueous solution. The dried granule is homogenised with 2776.0 g of amlodipine besilate, with 700.0 g of sodium starch glycolate and 24436.0 g of microcrystalline cellulose, and finally mixed with 400.0 g of magnesium stearate. The mixture is compressed into tablets of 0.2 g or the same quantity is filled into capsules.

| | |
|---|---|
| amlodipine besilate | 0.01388 g |
| lisinopril dihydrate | 0.00272 g |
| magnesium stearate | 0.00200 g |
| sodium starch glycolate | 0.00600 g |
| povidone | 0.00600 g |
| microcrystalline cellulose | 0.16940 g |
| | 0.20000 g |

The dissolution of the product meets the requirements of Ph.Eur.

### Example 5.

61.2 g of lisinopril dihydrate is mixed with 500.0 g of microcrystalline cellulose, then with 900.0 g of sodium starch glycolate, 10000.0 g of microcrystalline cellulose and 225.0 g of povidone. The mixture is granulated with 5000 g of water in a high shear granulator and vacuum dryer. (The polymer aqueous solution of 255.0 g of povidone can be alternatively used.) The wetted blend is dried under vacuum (1-500 mbar) until the initial moisture content is reached. After sizing a mixture of 156.15 g of amlodipine besilate and 500.0 g of microcrystalline cellulose is admixed and homogenization is completed with 31937.65 g of microcrystalline cellulose. Finally 675.0 g of magnesium stearate and 45.0 g of colloidal silicon dioxide are added and the mixture is compressed into tablets of 2 g weigt having the composition as follows:

| | |
|---|---|
| amlodipine besilate | 0.00694 g |
| lisinopril dihydrate | 0.00272 g |
| colloidal silicon dioxide | 0.00200 g |
| povidone | 0.01000 g |
| magnesium stearate | 0.03000 g |
| sodium starch glycolate | 0.04000 g |
| microcrystalline cellulose | 1.90834 g |
| | 2.00000 g |

The dissolution of the product meets the requirements of Ph.Eur.

### Example 6.

163.2 g of lisinopril dihydrate is mixed with 60.0 g of sodium starch glycolate and 893.0 g of microcrystalline cellulose and granulated with the polymer solution of 30.0 g of povidone and 500.0 g of purified water. The wetted bland is dried under vacuum (1-500 mbar) until the initial moisture content is reached. After sizing a mixture of 832.8 g of amlodipine besilate and 1000.0 g of microcrystalline cellulose is admixed. Finally 18.0 g of magnesium stearate and 3.0 g of colloidal silicon dioxide are added and the mixture is filled into capsules in 0.05 g quantities.

| | |
|---|---|
| amlodipine besilate | 0.01388 g |
| lisinopril dihydrate | 0.00272 g |
| colloidal silicon dioxide | 0.00005 g |
| magnesium stearate | 0.00030 g |
| povidone | 0.00050 g |
| sodium starch glycolate | 0.00100 g |
| microcrystalline cellulose | 0.03155 g |
| | 0.05000 g |

The dissolution of the product meets the requirements of Ph.Eur.

### Example 7.

2176.0 g of lisinopril dihydrate is mixed with 80.0 g of sodium starch glycolate and 172.0 g of microcrystalline cellulose and granulated with the polymer solution of 100.0 g of purified water and 15.0 g of povidone. The dried granule is sieved (0.8 mm mesh) and homogenised with 347.0 g of amlodipine besilate, 20.0 g of sodium starch glycolate and 160.0 g of microcrystalline cellulose. Finally 25.0 g of magnesium stearate and 5.0 g of colloidal silicon dioxide are added and 0.06 g quantities of the mixture are compressed into tablets or filled capsules.

| | |
|---|---|
| amlodipine besilate | 0.00694 g |
| lisinopril dihydrate | 0.04352 g |
| colloidal silicon dioxide | 0.00010 g |
| povidone | 0.00030 g |
| magnesium stearate | 0.00100 g |
| sodium starch glycolate | 0.00200 g |
| microcrystalline cellulose | 0.00614 g |
| | 0.06000 g |

The dissolution of the product meets the requirements of Ph.Eur.

## Claims

1. A pharmaceutical composition containing 0.3-30 wt %(m/m) of amlodipine hesitate and 0.1-75 wt %(m/m) of lisinopril dihydrate as active ingredients in admixture with one or more excipient(s) commonly used in the pharmaceutical industry with the proviso, that said excipient is other than a calcium salt of phosphoric acid.

2. The composition according to claim 1, herein the mass-mass ratio of amiodipine besilate and lisinopril dihydrate is 1:2 preferably 0.00694 g of amlodipine besilate and 0.01088 g of lisinopril dihydrate, are present.

3. The composition according to claim 1 or 2, which comprises 20-90 wt %(m/m) of microcrystalline cellulose, 2-8 wt %(m/m) of sodium starch glycolate and less than 5 wt %(m/m) of magnesium stearate as excipients.

4. A process for the preparation of a pharmaceutical composition as defined in claim 1, which comprises preparing a mixture of lisinopril dihydrate, sodium carboxymethyl amylopectin and microcrystalline cellulose with the absence of a calcium salt of phosphoric acid, granulating said mixture with water, drying the wet granulated mass in vacuo at a product temperature below 35-40 °C, homogenizing the dry blend obtained with amlodipine besilate, sodium carboxymethyl amylopectin and microcrystalline cellulose, then mixing the blend with magnesium stearate and compressing the mixture obtained into tablets or filling it into capsules.

5. A process according to claim 4, herein the drying step is aided with the use of dielectric energy.

## Patentansprüche

1. Pharmazeutische Zusammensetzung, die als wirksame Bestandteile 0,3 bis 30 Gew.% Amlodipinbesilat und 0,1 bis 75 Gew.% Lisinoprildihydrat in einer Mischung mit einem oder mehreren, in der pharmazeutischen Industrie gewöhnlich verwendeten, Hilfsstoff(en) enthält, vorausgesetzt, dass der Hilfsstoff ein anderer ist als ein Kalziumsalz von Phosphorsäure.

2. Zusammensetzung gemäß Anspruch 1, wobei das Massenverhältnis von Amlodipinbesilat und Lisinoprildihydrat 1:2 ist und vorzugsweise 0,00694 g Amlodipinbesilat und 0,01088 g Lisinoprildihydrat vorliegen.

3. Zusammensetzung gemäß Anspruch 1 oder 2, die 20 bis 90 Gew.%(m/m) mikrokristalline Cellulose, 2 bis 8 Gew.%(m/m) Natriumstärkeglycolat und weniger als 5 Gew.%(m/m) Magnesiumstearat als Hilfsstoffe umfasst.

4. Verfahren zur Herstellung einer pharmazeutischen Zusammensetzung gemäß Anspruch 1, welches Herstellen einer Mischung aus Lisinoprildihydrat, Natriumcarboxymethylamylopectin und mikrokristalliner Cellulose in Abwesenheit eines Kalziumsalzes von Phosphorsäure,
Granulieren der Mischung mit Wasser,
Trocknen der feuchten Granulatmasse im Vakuum bei einer Produkttemperatur unter 35 bis 40°C,
Homogenisieren der mit Amlodipinbesilat, Natriumcarboxymethylamylopectin und mikrokristalliner Cellulose erhaltenen Trockenmischung,
anschließend vermischen der Mischung mit Magnesiumstearat und
Verdichten der erhaltenen Mischung in Tabletten oder Füllen in Kapseln.

5. Verfahren gemäß Anspruch 4, wobei der Trocknungsschritt durch Verwendung von dielektrischer Energie unterstützt wird.

## Revendications

1. Composition pharmaceutique contenant de 0,3 à 30 % en poids (m/m) de bésilate d'amlodipine et de 0,1 à 75 % en poids (m/m) de dihydrate de lisinopril en tant qu'ingrédients actifs en mélange avec un ou plusieurs excipients couramment utilisés dans l'industrie pharmaceutique à condition que ledit excipient ne soit pas un sel de calcium d'acide phosphorique.

2. Composition selon la revendication 1, dans laquelle le rapport masse-masse entre le bésilate d'amlodipine et le dihydrate de lisinopril est de 1 : 2, de préférence 0,00694 g de bésilate d'amlodipine et 0,01088 g de dihydrate de lisinopril sont présents.

3. Composition selon la revendication 1 ou 2, qui comprend de 20 à 90 % en poids (m/m) de cellulose microcristalline, de 2 à 8 % en poids (m/m) de glycolate d'amidon sodique et moins de 5 % en poids (m/m) de stéarate de magnésium en tant qu'excipients.

4. Procédé de préparation d'une composition pharmaceutique selon la revendication 1, qui comprend la préparation d'un mélange de dihydrate de lisinopril, de carboxyméthyl amylopectine sodique et de cellulose microcristalline en l'absence d'un sel de calcium d'acide phosphorique, la granulation dudit mélange avec de l'eau, le séchage de la masse granulée humide sous vide à une température de produit en dessous de 35 à 40 °C, l'homogénéisation du mélange sec obtenu avec le bésilate d'amlodipine, la carboxyméthyl amylopectine sodique et la cellulose microcristalline, puis le mélange du mélange avec du stéarate de magnésium et le compressage du mélange obtenu en comprimés ou le remplissage dans des capsules.

5. Procédé selon la revendication 4, dans lequel l'étape de séchage est facilitée par l'utilisation d'énergie diélectrique.
